# EUROPEAN PATENT APPLICATION

(11) **EP 4 442 811 A1**
(43) Date of publication of application: **09.10.2024**
(21) Application number: 22901124.2
(22) Date of filing: 18.11.2022
(51) Int. Cl.: C12N 5/074, A61K 8/49, A61K 8/9706, A61K 31/4166, A61K 36/02, A61K 45/00, A61P 17/00, A61P 43/00, A61Q 19/08

(54) **STEM CELL PROLIFERATION PROMOTER**

(30) Priority: 03.12.2021 JP 2021196698
(71) Applicant: Shiseido Company, Ltd., Chuo-ku Tokyo 104-0061 (JP)
(72) Inventor: IRIYAMA, Shunsuke, Tokyo 104-0061 (JP)
(74) Representative: Cabinet Beau de Loménie
(86) International application number: PCT/JP2022/042858
(87) International publication number: WO 2023/100691

(57) **Abstract**

The present disclosure relates to providing a substance and a composition that promote the proliferation of epidermal stem cells and are effective in improving wrinkles and barrier function from the viewpoint of maintaining or improving quality of life (QOL) in terms of both health and beauty. More specifically, a composition including 1-(2-hydroxyethyl)-2-imidazolidinone (HEI) or a derivative thereof and a seaweed extract such as Marinwort IPC-12 AGS can be used to promote the proliferation of epidermal stem cells and obtain a beneficial effect on wrinkles and barrier function.

## Description

### FIELD

The present invention relates to an agent for promoting proliferation of epidermal stem cells and to its use, and more specifically it relates to an agent for promoting proliferation of epidermal stem cells comprising a heparanase/MMP-9 inhibitor and a seaweed extract, and to its use.

### BACKGROUND

In light of future prospects of an ever increasingly aging society, research and development on the mechanisms of aging are being carried out with increasing avidity. Skin forms the outermost layer of the human body, and it not only performs an important role for bodily protection but is also an important aspect of one's visual appearance, making it a particularly important organ from a cosmetic viewpoint. There has been increasing interest in preventing skin aging from the viewpoint of maintaining or improving quality of life (QOL) in terms of both health and beauty.

The epidermis is a skin tissue type present on the outermost layer of skin. The epidermis is composed mainly of the stratum corneum, granular layer, stratum spinosum and basal lamina. Basal cells in the basal lamina divide and migrate to the outer layer. During the course of migration, these cells become enucleated and flattened, while differentiating to the stratum corneum, with the stratum corneum eventually sloughing off. The turnover period is said to last about 45 days. Senescent skin, however, has a slower turnover rate, such that the epidermis becomes thinner as a whole. This is known to result in loss of skin function, including reduced barrier function and lower moisture content. Basal cells are highly divisible but do not undergo unlimited repeated division, dividing a fixed number of times and then failing to divide further. Basal cells are newly supplied by differentiation of a subset of the epidermal basal stem cells residing on the basal membrane (also referred to herein simply as "epidermal stem cells"). However, the number of epidermal basal stem cells is known to decrease with senescence, and when the number of epidermal basal stem cells decreases, signs of aging appear such as thinning of the epidermis, drying of the epidermis and reduction in its barrier function.

NPL 1 discloses that photoaging-induced reduction in laminin-511 in the basal membrane causes decreased epidermal stem cells/precursor cells thereof, and further that HEI (hydroxyethyl-imidazolidinone) has an effect of maintaining epidermal stem cell counts.

### [CITATION LIST]

### [NON PATENT LITERATURE]

[NPL 1] Shunsuke Iriyama et al., Scientific Reports, 10(1): 12592, 2020.

### SUMMARY

### [TECHNICAL PROBLEM]

The object of the present invention is the discovery of a substance and composition that are effective for increasing epidermal stem cell counts, in order to improve skin wrinkles and barrier function from the viewpoint of supporting or enhancing quality of life (QOL) in terms of both health and beauty.

### [SOLUTION TO PROBLEM]

As a result of avid research by the present inventors, it was found that a combination of a heparanase/MMP-9 inhibitor such as 1-(2-hydroxyethyl)-2-imidazolidinone or derivative thereof, with a seaweed extract such as a mixed extract of brown algae, red algae and green algae, or Argelex, exhibits an effect of increasing the numbers of epidermal stem cells.

The present disclosure thus provides the following aspects.
(1) An agent for promoting proliferation of epidermal stem cells, comprising:
   a heparanase/MMP-9 inhibitor and
   a seaweed extract
   as active ingredients.
(2) The agent according to aspect 1 wherein the heparanase/MMP-9 inhibitor is 1-(2-hydroxyethyl)-2-imidazolidinone or derivative thereof.
(3) The agent according to aspect 2, wherein the 1-(2-hydroxyethyl)-2-imidazolidinone or derivative thereof is a cyclic carboxamide derivative represented by the following general formula (I): 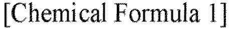 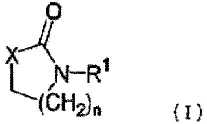 (where n is an integer of 1 to 3, R¹ is a hydrogen atom or a hydrocarbon group having 1 to 6 carbon atoms optionally substituted with a hydroxyl group, X is a group represented by -CH₂- or -N(R²)-, and R² is a hydrogen atom or a hydrocarbon group having 1 to 6 carbon atoms optionally substituted with a hydroxyl group).
(4) The agent according to any one of aspects 1 to 3, wherein the seaweed extract is a mixed extract of brown algae, red algae and green algae, or Argelex.
(5) The agent according to any one of aspects 1 to 4, wherein the heparanase/MMP-9 inhibitor is 1-(2-hydroxyethyl)-2-imidazolidinone and the seaweed extract is Argelex.
(6) An agent for improvement of wrinkles, comprising:
   a heparanase/MMP-9 inhibitor and
   a seaweed extract
   as active ingredients.
(7) An agent for improvement of skin barrier function, comprising:
   a heparanase/MMP-9 inhibitor and
   a seaweed extract
   as active ingredients.
(8) A cosmetic method comprising applying a composition comprising a heparanase/MMP-9 inhibitor and a seaweed extract, onto skin.
(9) A non-therapeutic method for prevention or improvement of wrinkles, comprising applying a composition comprising a heparanase/MMP-9 inhibitor and a seaweed extract, onto skin.
(10) A non-therapeutic method for improvement of skin barrier function, comprising applying a composition comprising a heparanase/MMP-9 inhibitor and a seaweed extract, onto skin.

### [ADVANTAGEOUS EFFECTS OF INVENTION]

Since the agent of the present disclosure not only maintains but also increases epidermal stem cell counts, it can be suitably used as a cosmetic composition, such as a cosmetic product, that is effective for improving wrinkles and barrier function.

### BRIEF DESCRIPTION OF DRAWINGS

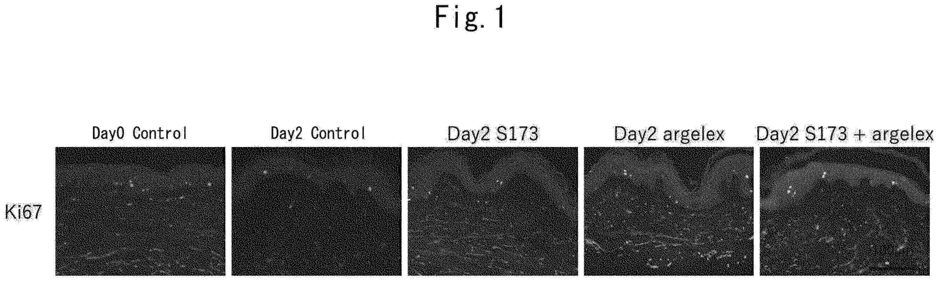

Fig. 1 is a set of images showing the results of examining the effects of S173 (HEI), Argelex (Argelex) and a combination of S173 and Argelex on epidermal stem cells, based on Ki67 expression. DayO control is a control untreated before culturing, Day2 control is a control treated only by culturing for 2 days, S173 is a specimen treated with S173 alone, Argelex is a specimen treated with Argelex alone, and S173+Argelex is a specimen treated with a combination of S173 and Argelex.

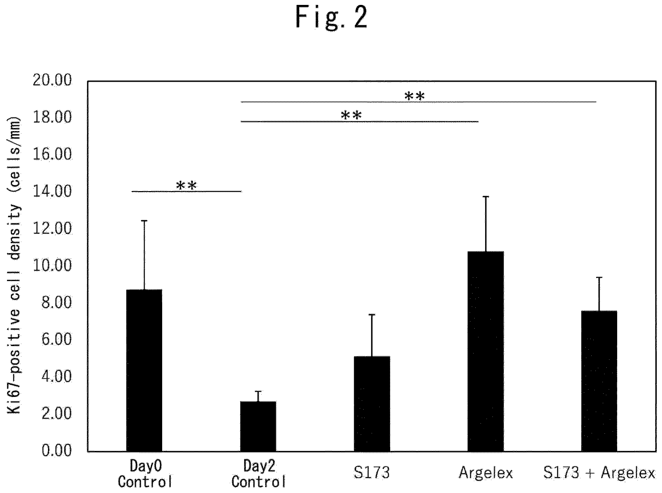

Fig. 2 is a graph showing the results of Fig. 1. The ordinate represents Ki67-positive cell density. On the abscissa, from left are DayO control: a control untreated before culturing, Day2 control: a control treated only by culturing for 2 days, S173: a specimen treated with S173 alone, Argelex: a specimen treated with Argelex alone, and S173+Argelex: a specimen treated with a combination of S173 and Argelex. The values are mean and SD for three independent experiments. Statistical analysis was carried out using one-way analysis of variance and Turkey multiple comparison test.

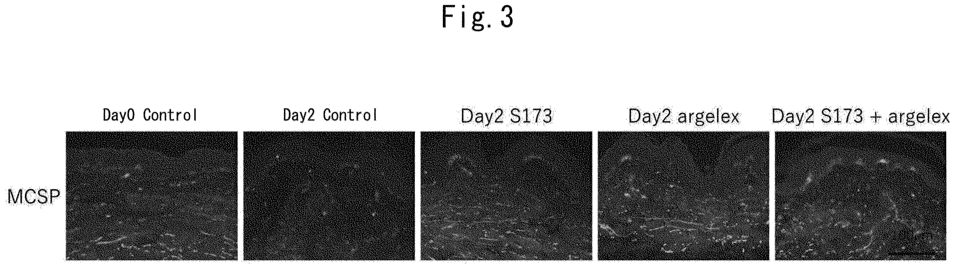

Fig. 3 is a set of images showing the results of examining the effects of S173 (HEI), Argelex (Argelex) and a combination of S173 and Argelex on epidermal stem cells, based on MCSP expression. DayO control is a control untreated before culturing, Day2 control is a control treated only by culturing for 2 days, S173 is a specimen treated with S173 alone, Argelex is a specimen treated with Argelex alone, and S173+Argelex is a specimen treated with a combination of S173 and Argelex.

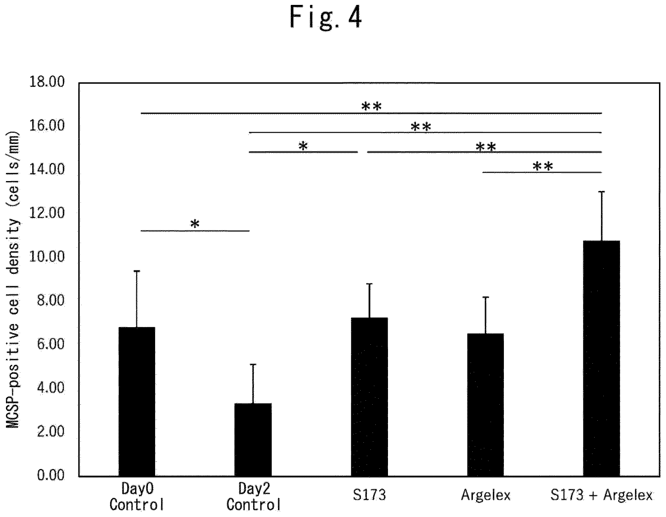

Fig. 4 is a graph showing the results of Fig. 3. The ordinate represents MCSP-positive cell density. On the abscissa, from left are DayO control (leftmost Cont): a control untreated before culturing, Day2 control (second Cont from left): a control treated only by culturing for 2 days, S173: a specimen treated with S173 alone, Argelex: a specimen treated with Argelex alone, and S173+Argelex: a specimen treated with a combination of S173 and Argelex. The values are mean and SD for three independent experiments. Statistical analysis was carried out using one-way analysis of variance and Turkey multiple comparison test.

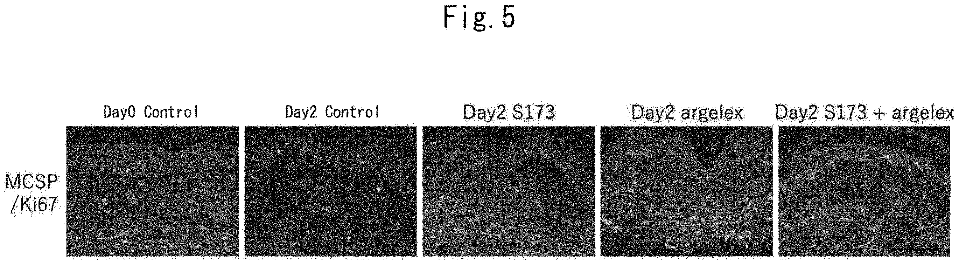

Fig. 5 is a set of images showing the results of examining the effects of S173 (HEI), Argelex (Argelex) and a combination of S173 and Argelex on epidermal stem cells, based on Ki67 and MCSP coexpression. DayO control is a control untreated before culturing, Day2 control is a control treated only by culturing for 2 days, S173 is a specimen treated with S173 alone, Argelex is a specimen treated with Argelex alone, and S173+Argelex is a specimen treated with a combination of S173 and Argelex.

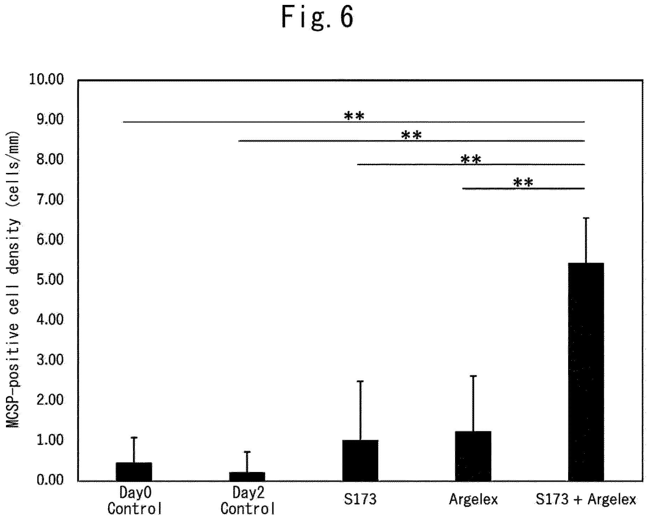

Fig. 6 is a graph showing the results of Fig. 5. The ordinate represents Ki67+MCSP-positive cell density. On the abscissa, from left are DayO control (leftmost Cont): a control untreated before culturing, Day2 control (second Cont from left): a control treated only by culturing for 2 days, S 173: a specimen treated with S 173 alone, Argelex: a specimen treated with Argelex alone, and S 173+Argelex: a specimen treated with a combination of S 173 and Argelex. The values are mean and SD for three independent experiments. Statistical analysis was carried out using one-way analysis of variance and Turkey multiple comparison test.

### DESCRIPTION OF EMBODIMENTS

### Agent for promoting proliferation of epidermal stem cells

One aspect of the disclosure relates to an agent for promoting proliferation of epidermal stem cells, comprising a heparanase/MMP-9 inhibitor and a seaweed extract as active ingredients.

According to one aspect, the heparanase/MMP-9 inhibitor to be used in the agent for promoting proliferation of epidermal stem cells of the disclosure may be a mixture of a heparanase inhibitor and an MMP-9 inhibitor, but it is preferably a bifunctional compound with both heparanase inhibition activity and MMP-9 inhibition activity. According to this aspect, therefore, 1-(2-hydroxyethyl)-2-imidazolidinone (HEI) or derivative thereof having both heparanase inhibition activity and MMP-9 inhibition activity may be used as one active ingredient in the agent for promoting proliferation of epidermal stem cells of the disclosure. The compound 1-(2-hydroxyethyl)-2-imidazolidinone (HEI) may be referred to as "S-173" or "S173" throughout the present specification.

The compound 1-(2-hydroxyethyl)-2-imidazolidinone (HEI) has the following structure.

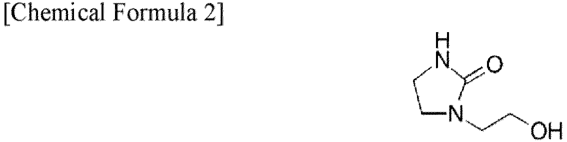

A derivative of 1-(2-hydroxyethyl)-2-imidazolidinone may be a compound represented by the following general formula (I): 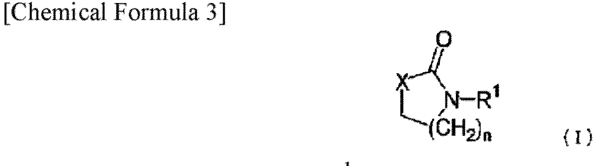 (where n is an integer of 1 to 3, R¹ is a hydrogen atom or a hydrocarbon group having 1 to 6 carbon atoms optionally substituted with a hydroxyl group, X is a group represented by -CH₂- or -N(R²)-, and R² is a hydrogen atom or a hydrocarbon group having 1 to 6 carbon atoms optionally substituted with a hydroxyl group).

According to one aspect, the seaweed extract used in the agent for promoting proliferation of epidermal stem cells of the disclosure is an extract obtained from at least one type of algae selected from the group consisting of brown algae, red algae and green algae. According to a more preferred mode, the seaweed extract is extract from brown algae, red algae and green algae. More preferably, it is extract of brown algae such as *Laminaria* and *Undaria,* red algae such as *Grateloupia,* and green algae such *Ulva,* and most preferably Argelex. The solvent used may be any desired solvent, such as water, alcohol, an ether or an ester, either alone or in admixture. An alcohol that is used may be a monohydric alcohol such as methanol, ethanol, propanol or butanol, a dihydric alcohol such as ethylene glycol, propylene glycol or butylene glycol, or a trihydric alcohol such as glycerin. Ethers that may be used include dimethyl ether, diethyl ether, ethyl methyl ether and tetrahydrofuran. Esters that may be used include methyl acetate and ethyl acetate. When these are used as a mixture, they may be used in any desired mixing ratio. For example, a liquid mixture of water and 1,3-butylene glycol may be in a range of 1: 10 to 10: 1, and more preferably in a range of 3:10 to 10:3. A 1:1 liquid mixture may also be used. The seaweed extract may be formulated into a cosmetic. For example, an amount of 0.0001% to 10% and preferably 0.001% to 1.0% of seaweed extract may be added to a cosmetic. An example may be addition of 0.1% seaweed extract to a cosmetic such as an essence, cosmetic water, emulsion or cream, for example.

According to one aspect, the seaweed extract used in the agent for promoting proliferation of epidermal stem cells of the disclosure may be Argelex. Argelex is a cosmetic material marketed by Ichimaru Pharcos Co., Ltd., and is a seaweed extract. More specifically, Argelex is a mixed extract from brown algae, red algae and green algae. It is obtained by mixing brown algae extract, obtained by immersing total brown algae in 50% 1,3-butylene glycol for 3 days and filtering, with extract of brown algae, red algae and green algae obtained by immersing total brown algae, red algae and green algae in 50% 1,3-butylene glycol for 3 days and filtering it. Argelex is thought to improve moisture content and exhibit an inhibiting effect against skin roughening. The brown algae used in Argelex are *Laminaria* and *Undaria* algae, examples of which are narrow-leaved tangle and wakame. The red algae used in Argelex are *Grateloupia* algae, examples of which are *Eucheuma serra* and *Grateloupia sparsa.* The green algae used in Argelex are *Ulva* algae, an example of which is *Ulva linza.*

According to one aspect, the agent for promoting proliferation of epidermal stem cells of the disclosure includes a combination of 1-(2-hydroxyethyl)-2-imidazolidinone and Argelex.

Stated differently, the agent for promoting proliferation of epidermal stem cells of the disclosure can be considered to be a composition for promoting proliferation of epidermal stem cells which comprises a heparanase/MMP-9 inhibitor such as 1-(2-hydroxyethyl)-2-imidazolidinone and a seaweed extract such as Argelex. The composition can be used for non-therapeutic cosmetic purposes, in particular. From a different viewpoint, one aspect of the disclosure relates to the use of a heparanase/MMP-9 inhibitor such as 1-(2-hydroxyethyl)-2-imidazolidinone and a seaweed extract such as Argelex for promoting proliferation of epidermal stem cells. For such usage, the heparanase/MMP-9 inhibitor and a seaweed extract may be used in the form of a composition also incorporating other components. From a different viewpoint, one aspect of the disclosure relates to the use of a heparanase/MMP-9 inhibitor such as 1-(2-hydroxyethyl)-2-imidazolidinone and a seaweed extract such as Argelex for production of an agent for promoting proliferation of epidermal stem cells. From yet a different viewpoint, one aspect of the disclosure relates to a method for promoting proliferation of epidermal stem cells which includes administration of a heparanase/MMP-9 inhibitor such as 1-(2-hydroxyethyl)-2-imidazolidinone and a seaweed extract such as Argelex to a subject (such as a human).

### Agent for improvement of wrinkles

One aspect of the disclosure relates to an agent for improvement of wrinkles, comprising a heparanase/MMP-9 inhibitor and a seaweed extract as active ingredients. Promoting proliferation of epidermal stem cells improves the supply of epidermal cells, leading to improvement of wrinkles. The wrinkles may be fine wrinkles and/or large wrinkles. Improvement of fine wrinkles may be improvement of epidermal wrinkles, as epidermal proliferation is promoted. Improvement of large wrinkles may be improvement of wrinkles as papillary layer collagen production is promoted due to accelerated production of PDGF-BB secreted from the epidermis. The heparanase/MMP-9 inhibitor and seaweed extract were explained in detail above. According to one aspect, the agent for improvement of wrinkles of the disclosure includes a combination of 1-(2-hydroxyethyl)-2-imidazolidinone and Argelex. Improvement of wrinkles can be evaluated using any desired method such as, for example, replica analysis, grade assessment or visual assessment based on a photograph, or using an image analyzer.

Stated differently, the agent for improvement of wrinkles of the disclosure can be considered to be a composition for improvement of wrinkles which comprises a heparanase/MMP-9 inhibitor such as 1-(2-hydroxyethyl)-2-imidazolidinone and a seaweed extract such as Argelex. The composition can be used for non-therapeutic cosmetic purposes, in particular. From a different viewpoint, one aspect of the disclosure relates to the use of a heparanase/MMP-9 inhibitor such as 1-(2-hydroxyethyl)-2-imidazolidinone and a seaweed extract such as Argelex for improvement of wrinkles. For such usage, the heparanase/MMP-9 inhibitor and seaweed extract may be used in the form of a composition also incorporating other components. From yet a different viewpoint, one aspect of the disclosure relates to the use of a heparanase/MMP-9 inhibitor such as 1-(2-hydroxyethyl)-2-imidazolidinone and a seaweed extract such as Argelex for production of a agent for improvement of wrinkles. From yet a different viewpoint, one aspect of the disclosure relates to a method for improvement of wrinkles which includes administration of a heparanase/MMP-9 inhibitor such as 1-(2-hydroxyethyl)-2-imidazolidinone and a seaweed extract such as Argelex to a subject (such as a human).

### Agent for improvement of skin barrier function

One aspect of the disclosure relates to an agent for improvement of skin barrier function (an agent for promoting restoration of skin barrier) comprising a heparanase/MMP-9 inhibitor and a seaweed extract as active ingredients. By promoting proliferation of epidermal stem cells, turnover is improved, resulting in faster recovery of the skin barrier and improved skin barrier function. The heparanase/MMP-9 inhibitor and seaweed extract were explained in detail above. According to one aspect, the agent for improvement of skin barrier function of the disclosure includes a combination of 1-(2-hydroxyethyl)-2-imidazolidinone and Argelex. Skin barrier function can be evaluated based on indicators such as transepidermal water loss (TEWL) or the degree of penetration into skin of an indicator substance such as a pigment, for example. TEWL of skin can be measured using a device such as a VapoMeter.

Stated differently, the agent for improvement of skin barrier function of the disclosure can be considered to be a composition for improvement of barrier function which comprises a heparanase/MMP-9 inhibitor such as 1-(2-hydroxyethyl)-2-imidazolidinone and a seaweed extract such as Argelex. The composition can be used for non-therapeutic cosmetic purposes, in particular. From a different viewpoint, one aspect of the disclosure relates to the use of a heparanase/MMP-9 inhibitor such as 1-(2-hydroxyethyl)-2-imidazolidinone and a seaweed extract such as Argelex for improvement of barrier function. For such usage, the heparanase/MMP-9 inhibitor and seaweed extract may be used in the form of a composition also incorporating other components. From yet a different viewpoint, one aspect of the disclosure relates to the use of a heparanase/MMP-9 inhibitor such as 1-(2-hydroxyethyl)-2-imidazolidinone and a seaweed extract such as Argelex for production of an agent for improvement of skin barrier function. From yet a different viewpoint, one aspect of the disclosure relates to a method for improvement of barrier function which includes administration of a heparanase/MMP-9 inhibitor such as 1-(2-hydroxyethyl)-2-imidazolidinone and a seaweed extract such as Argelex to a subject (such as a human).

### Preparation

The 1-(2-hydroxyethyl)-2-imidazolidinone or derivative thereof can be synthesized by a publicly known method, or a commercial product may easily be purchased. The seaweed extract may also be extracted by a publicly known method, or a commercial product may easily be purchased.

The 1-(2-hydroxyethyl)-2-imidazolidinone or derivative thereof may also be prepared as an inorganic salt or organic salt by a publicly known method. There are no particular restrictions on salts to be used for this aspect of the disclosure, and examples include inorganic salts such as hydrochlorides, sulfates, phosphates, hydrobromates sodium salts, potassium salts, magnesium salts, calcium salts and ammonium salts. Organic salts include acetates, lactates, maleates, fumarates, tartrates, citrates, methanesulfonates, *p*-toluenesulfonates, triethanolamine salts, diethanolamine salts and amino acid salts.

The heparanase/MMP-9 inhibitor used in the agent of the disclosure is preferably 1-(2-hydroxyethyl)-2-imidazolidinone or derivative thereof having a heparanase activity inhibiting effect or MMP-9 inhibiting effect, and especially one having both a heparanase activity inhibiting effect and an MMP-9 inhibiting effect. The agent of the disclosure may include only one type of 1-(2-hydroxyethyl)-2-imidazolidinone or derivative thereof, or it may include two or more of such compounds or salts thereof in any combination and proportion. The agent of the disclosure may also include only a single type of seaweed extract such as Argelex, or it may include two or more seaweed extracts in any combination and proportion.

The content of the 1-(2-hydroxyethyl)-2-imidazolidinone or derivative or salt thereof in the agent of the disclosure is not particularly restricted so long as it is a sufficient amount to adequately exhibit the desired effect, and it may be selected as appropriate according to the purpose of use of the agent. Generally, however, the proportion of the 1-(2-hydroxyethyl)-2-imidazolidinone or derivative or salt thereof with respect to the total agent is preferred to be 0.0001 mass% or greater and more preferably 0.001 mass% or greater, and 5 mass% or lower and more preferably 2.0 mass% or lower (such as 1.5 mass%). When two or more types of the 1-(2-hydroxyethyl)-2-imidazolidinone or derivative or salt thereof are used, the total amount should satisfy the range specified above. The content of the seaweed extract in the agent of the disclosure is likewise not particularly restricted so long as it is a sufficient amount to adequately exhibit the desired effect, and it may be selected as appropriate according to the purpose of use of the agent. Generally, however, the proportion of the seaweed extract with respect to the total agent is preferred to be 0.0001 mass% or greater and more preferably 0.001 mass% or greater, and 5 mass% or lower and more preferably 2.0 mass% or lower (such as 1.5 mass%). When two or more different seaweed extracts are used, their total amount should satisfy the range specified above.

The agent of the disclosure may comprise any other components in addition to the heparanase/MMP-9 inhibitor and seaweed extract, so long as they essentially do not impair the function of the combination of the heparanase/MMP-9 inhibitor and seaweed extract. Other components include other compounds with inhibiting action against heparanase activity or other activity (especially MMP-9 inhibition activity) (such as CGS27023A and BIPBIPU), and medically acceptable carriers and/or adjuvants. Examples of other components include, but are not limited to, herbal medicines that act as heparanase inhibitors, such as valerian (Kanokoso) extract, lily extract, *Peucedanum japonicum* (Choumeisou) extract, *Sapindus mukorossi* (Mukuroji) extract and citrus unshiu peel extract (see Japanese Patent Application No. 2014-165586), and herbal medicines that act as MMP inhibitors, such as mangosteen extract, turmeric extract and tormentilla extract (see Japanese Patent Application No. 2018-545781). Such other components may be used alone, or 2 or more may be used in any desired combination and ratio. One aspect of the invention, therefore, relates to agents such as an agent for promoting proliferation of epidermal stem cells and agent for improvement of wrinkles, comprising a compound having both heparanase inhibition activity and MMP-9 inhibition activity and/or a combination of a heparanase inhibitor and MMP-9 inhibitor, and a seaweed extract as active ingredients.

The agent of the disclosure may be produced by a common method, and as a component for an external preparation for skin, a single type of each component (the heparanase/MMP-9 inhibitor and seaweed extract), or 2 or more different ones, may be prepared separately, optionally with addition of components commonly used in external preparations for skin as necessary, such as quasi drug-containing cosmetics or pharmaceuticals, examples of which include oils, surfactants, powders, coloring materials, water, alcohols, thickeners, chelating agents, silicones, antioxidants, ultraviolet absorbers, humectants, aromatics, various drug components, antiseptic agents, pH regulators and neutralizers.

The route of administration and dosage form of the agent of the disclosure are not restricted, and may be selected as appropriate for the purpose. Examples of routes of administration include local administration (such as topical skin aaplication), oral administration and parenteral administration (such as intravenous administration and intraperitoneal administration), but an external preparation for skin is preferred for use as an agent of the disclosure, such as an agent for promoting proliferation of epidermal stem cells. The dosage form, for local administration (a topical skin application material), may be a form in which a solution system, solubilized system, emulsified system, powder-dispersed system, water/oil two-layer system, water/oil/powder three-layer system or the like, is prepared as a patch, ointment, cream, emulsion, cosmetic water, gel or aerosol. For oral administration, it may be in the form of a solid formulation, such as a tablet, coated tablet, sugar-coated tablet, granules, powder, capsule (for example, a hard or soft gelatin capsule), or a liquid formulation (solution or suspension) such as an internal liquid drug or syrup. For parenteral administration, it may be in the form of an injection or the like.

The agent of the disclosure may also comprise any one or more other components such as carriers and/or adjuvants in addition to the heparanase/MMP-9 inhibitor and seaweed extract of the disclosure so long as they essentially do not impair the function of the heparanase/MMP-9 inhibitor and seaweed extract. There are no particular restrictions on such other components, and they may be appropriately selected according to the purpose, dosage form and manner of administration of the medical composition, but medically acceptable carriers and/or adjuvants may be mentioned as examples. Examples of adjuvants include diluents, binders, disintegrators, thickeners, dispersing agents, reabsorption accelerators, taste correctives, buffering agents, surfactants, dissolving aids, preservatives, emulsifiers, isotonizing agents, stabilizers and pH regulators.

As specific examples, when the agent of the disclosure is to be used as an external preparation for skin, components commonly used in external preparations, such as skin whiteners, humectants, antioxidants, oil components, ultraviolet absorbers, surfactants, thickeners, alcohols, powder constituents, coloring agents, aqueous components, water or various skin nutrient preparations, may be appropriately added as necessary. In addition, there may also be added appropriate amounts of metal ion chelators such as disodium edetate, trisodium edetate, sodium citrate, sodium polyphosphate, sodium metaphosphate or gluconic acid, antiseptic agents such as methylparaben, ethylparaben or butylparaben, or caffeine, tannins, verapamil, tranexamic acid or its derivatives, licorice extract, drug agents such as glabridin, Chinese quince fruit hot water extract, herbal medicines, tocopherol acetate, glycyrrhizic acid and its derivatives or salts, skin whiteners such as vitamin C, magnesium ascorbate phosphate, glucoside ascorbate, arbutin or kojic acid, saccharides such as glucose, fructose, mannose, sucrose or trehalose, vitamin A derivatives such as retinoic acid, retinol, retinol acetate or retinol palmitate, or niacin derivatives such as amide nicotinate.

However, these components are not limited to those mentioned above. Such components may also be used in appropriate mixtures, according to formulations for prescribed dosage forms.

The dosage form of the external preparation for skin of the disclosure is not particularly restricted, and for example, it may be in any desired form such as a solution system, a solubilized system, an emulsified system, a powder-dispersed system, a water-oil two-layer system, a water-oil-powder three-layer system, an ointment, gel, aerosol, or the like. The form of use is also not particularly restricted, and for example, any desired form such as cosmetic water or an emulsion, cream, essence, jelly, gel, ointment, pack, mask, foundation or the like may be employed.

The agent of the disclosure can be applied onto skin for use in a cosmetic or non-therapeutic method intended to prevent formation of wrinkles and/or to alleviate or eliminate formed wrinkles. One aspect of the disclosure, therefore, relates to a cosmetic or non-therapeutic method in which a treatment-effective dose of a composition comprising a heparanase/MMP-9 inhibitor and a seaweed extract is applied onto skin of a subject (such as a human) in need of such treatment. The method of usage and dosage for an external preparation for skin according to the disclosure to be used in the cosmetic or non-therapeutic method is also not particularly restricted and may be appropriately established depending on the dosage form or the condition of the wrinkles to be treated, but typically a suitable dose, e.g. from 0.1 ml to 1 ml per cm² is rubbed directly onto the skin or absorbed in gauze and applied onto the skin, several (1-5) times per day. Use of the cosmetic or non-therapeutic method of the disclosure does not fall within the concept of "medical practice".

The present invention has been explained with concrete examples, but with the understanding that these are merely for illustration and that the invention may incorporate any desired modifications that fall within the scope of the claims of the invention. The features and aspects of the invention as laid out herein may also be applied to other modes incorporating various suitable or necessary modifications. Therefore, a feature specified by any one aspect may be combined with a function specified by any another aspect as appropriate. All patents, patent applications, scientific papers and textbooks, as well as references cited herein including their sequence accession numbers, and their cited references, are incorporated in their entirety by reference. In the case where one or more incorporated documents or similar materials differ from or contradict the present application in, but not exclusive to, the definition of a term, the use of a term or the description of a technical method, the description in the present application takes precedence.

The present invention will now be explained in greater detail by Examples, with the understanding that the invention is not meant to be limited to these Examples.

### EXAMPLES

### Culturing of fresh human skin

Fresh skin samples taken from the abdominal regions of participants (aged 20 to 30) with informed consent were purchased from KAC Co., Ltd. The samples were cultured in medium comprising an equal mixture of 10% FBS-DMEM (Thermo Fisher Scientific K.K., 11885084) and Humedia-KG2 (KURABO Industries Ltd., KK-2150S) with addition of 1-(2-hydroxyethyl)-2-imidazolidinone ("S173", final concentration: 0.01%), which has an effect of inhibiting both MMP-9 and heparanase, Argelex ("Argelex", final concentration: 0.01%), or a combination of 1-(2-hydroxyethyl)-2-imidazolidinone and Argelex ("S173+Argelex", final concentration: 0.01%). As a control, culturing was carried out in medium containing an equal amount of DMSO solvent lacking these agents. Medium was exchanged daily, and skin tissue slices were collected on the 2nd day (Day2).

### Paraffin blocking and slice preparation

Following the AMeX method, fresh human skin was dehydrated and fixed using cold acetone and exchanged with acetone, methyl benzoate and xylene in that order, and then embedded in paraffin. Slices with thicknesses of 3 µm were prepared for tissue staining.

### Immunostaining

Paraffin slices cut to 3 µm thicknesses were deparaffinized with xylene and hydrated using EtOH. Antibodies for Ki-67(SP6) (Thermo Fisher Scientific K.K., RM-9106-S, rabbit mouse monoclonal antibody) and antibodies for MCSP (melanoma-associated chondroitin sulphate proteoglycan) (Millipore, MAB2029, 9.2.27, mouse monoclonal antibody) were used for fluorescent immunostaining.

### Examining synergistic effect of S173 (HEI) and Argelex (Argelex)

Fig. 1 and Fig. 2 show the results of examining the effects of S173 (HEI), Argelex (Argelex) and a combination of S173 and Argelex on epidermal stem cells, based on Ki67 expression. Ki-67 is known to function as a marker for cell proliferation. As clearly seen in the graph of Fig. 2, the number of Ki67-positive cells increased by treatment with S173 (HEI), Argelex (Argelex) and the combination of S173 and Argelex, compared to the control without addition of agent (Day2 control), thus demonstrating that S173 (HEI) and Argelex both promote cell proliferation. Proliferative cells decrease after culturing for 2 days in this manner, but the number of proliferative cells increased with Argelex, being restored to the level of DayO.

Fig. 3 and Fig. 4 show the results of examining the effects of S173 (HEI), Argelex (Argelex) and the combination of S173 and Argelex on epidermal stem cells, based on MCSP expression. MCSP is known to function as an epidermal basal stem cell marker. As clearly seen in the graph of Fig. 4, the number of MCSP-positive cells increased by treatment with S173 (HEI), Argelex (Argelex) and the combination of S173 and Argelex, compared to the control without addition of agent (Day2 control), thus demonstrating that S173 (HEI) and Argelex both promote increase in epidermal stem cells. Stem cells decrease after culturing for 2 days in this manner, but the number of stem cells was maintained at the level of DayO with Argelex and S173, whereas with Argelex+S173 the number of stem cells increased to above the level of DayO. Since the number of stem cells was merely maintained at the level of the untreated control when using Argelex or S173 alone, the increased number of stem cells with the combination of Argelex and S173 was a completely unexpected and surprising result.

Fig. 5 and Fig. 6 show the results of examining the effects of S173 (HEI), Argelex (Argelex) and the combination of S173 and Argelex on epidermal stem cells, based on coexpression of Ki67 and MCSP. As clearly seen in the graph of Fig. 6, the number of Ki67+MCSP-positive cells increased by treatment with S173 (HEI), Argelex (Argelex) and the combination of S173 and Argelex, compared to the control, thus demonstrating that S173 (HEI) and Argelex both promote increase in proliferative epidermal stem cells. Moreover, the number of proliferative stem cells was shown to significantly increase with the combination of Argelex and S 173. This result, whereby the combination of Argelex and S173 not only suppressed reduction in epidermal stem cells to maintain the number of cells, but also promoted proliferation of epidermal stem cells to increase the number of stem cells, as a synergistic effect, was a completely unexpected and surprising result.

### References

1) Pan W, Miao HQ, Xu YJ, Navarro EC, Tonra JR, Corcoran E, et al. 1-[4-(1H-Benzoimidazol-2-yl)-phenyl]-3-[4-(1H-benzoimidazol-2-yl)-phenyl]-urea derivatives as small molecule heparanase inhibitors. Bioorganic & Medicinal Chemistry Letters. 2006; 16(2):409-12.
2) MacPherson LJ, Bayburt EK, Capparelli MP, Carroll BJ, Goldstein R, Justice MR, et al. Discovery of CGS 27023A, a non-peptidic, potent and orally active stromelysin inhibitor that blocks cartilage degradation in rabbits. Journal of Medicinal Chemistry. 1997; 40(16):2525-32.
3) Iriyama S, Yamanishi H, Kunizawa N, Hirao T, Amano S. 1-(2-Hydroxyethyl)-2-imidazolidinone, a heparanase and matrix metalloproteinase inhibitor, improves epidermal basement membrane structure and epidermal barrier function. Experimental Dermatology. 2019; 28(3):247-53.
4) Iriyama S, Yasuda M, Nishikawa S, Takai E, Hosoi J, Amano S. Decrease of laminin-511 in the basement membrane due to photoaging reduces epidermal stem/progenitor cells. Scientific Reports. 2020; 10(1): 12592.
5) Farhad Behzad, Paul E C Brenchley. A multiwell format assay for heparinase. Anal. Biochem. 2003 Sep 15; 320(2):207-13.

### INDUSTRIAL APPLICABILITY

Since the effect of the agent of the disclosure is not one of supporting epidermal stem cell counts but rather promoting their proliferation to increase cell counts, it can be suitably used as a cosmetic composition, such as a cosmetic product, that is effective for ameliorating wrinkles (improving epidermal fine wrinkles and large wrinkles in the dermal matrix), as well as promoting recovery of the epidermal barrier.

## Claims

1. An agent for promoting proliferation of epidermal stem cells, comprising:
a heparanase/MMP-9 inhibitor and
a seaweed extract
as active ingredients.

2. The agent according to claim 1 wherein the heparanase/MMP-9 inhibitor is 1-(2-hydroxyethyl)-2-imidazolidinone or a derivative thereof.

3. The agent according to claim 2, wherein the 1-(2-hydroxyethyl)-2-imidazolidinone or derivative thereof is a cyclic carboxamide derivative represented by the following general formula (I): 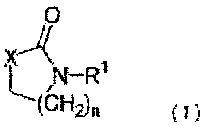 (where n is an integer of 1 to 3, R¹ is a hydrogen atom or a hydrocarbon group having 1 to 6 carbon atoms optionally substituted with a hydroxyl group, X is a group represented by -CH₂- or -N(R²)-, and R² is a hydrogen atom or a hydrocarbon group having 1 to 6 carbon atoms optionally substituted with a hydroxyl group).

4. The agent according to any one of claims 1 to 3, wherein the seaweed extract is a mixed extract of brown algae, red algae and green algae, or Argelex.

5. The agent according to any one of claims 1 to 4, wherein the heparanase/MMP-9 inhibitor is 1-(2-hydroxyethyl)-2-imidazolidinone and the seaweed extract is Argelex.

6. An agent for improvement of wrinkles, comprising:
a heparanase/MMP-9 inhibitor and
a seaweed extract
as active ingredients.

7. An agent for improvement of skin barrier function, comprising:
a heparanase/MMP-9 inhibitor and
a seaweed extract
as active ingredients.

8. A cosmetic method, comprising
applying a composition comprising a heparanase/MMP-9 inhibitor and a seaweed extract, onto skin.

9. A non-therapeutic method for prevention or improvement of wrinkles, comprising
applying a composition comprising a heparanase/MMP-9 inhibitor and a seaweed extract, onto skin.

10. A non-therapeutic method for improvement of skin barrier function, comprising
applying a composition comprising a heparanase/MMP-9 inhibitor and a seaweed extract, onto skin.
